(19) Europäisches Patentamt
European Patent Office
Office européen des brevets

(11) Veröffentlichungsnummer: **0 334 060 B1**

(12) **EUROPÄISCHE PATENTSCHRIFT**

(45) Veröffentlichungstag der Patentschrift: **23.06.93**

(51) Int. Cl.$^5$: **A61K 7/30**

(21) Anmeldenummer: **89103565.1**

(22) Anmeldetag: **01.03.89**

(54) **Reinigungstablette für Zahnprothesen.**

(30) Priorität: **19.03.88 DE 3809359**
**16.04.88 DE 3812693**

(43) Veröffentlichungstag der Anmeldung:
**27.09.89 Patentblatt 89/39**

(45) Bekanntmachung des Hinweises auf die Patenterteilung:
**23.06.93 Patentblatt 93/25**

(84) Benannte Vertragsstaaten:
**AT BE CH DE ES FR GB GR IT LI LU NL SE**

(56) Entgegenhaltungen:
**EP-A- 0 186 436**
**EP-A- 0 253 772**
**FR-A- 2 329 747**

(73) Patentinhaber: **RECKITT GMBH**
**Hoffmannstrasse 6**
**W-4920 Bad Salzuflen(DE)**

(72) Erfinder: **Reuss, Mira, Dr.**
**Nietzschestrasse 4**
**W-6800 Mannheim 1(DE)**

(74) Vertreter: **Goddar, Heinz J., Dr. et al**
**FORRESTER & BOEHMERT Franz-Joseph-Strasse 38**
**W-8000 München 40 (DE)**

EP 0 334 060 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Reinigungstablette zum selbsttätigen Reinigen von Zahnprothesen in wässeriger Lösung nach dem Oberbegriff des Hauptanspruches.

Reinigungstabletten für Zahnprothesen, wie sie z. B. in der DE-OS 21 33 710 beschrieben sind, werden in der Weise verwendet, daß sie zugleich mit der zu reinigenden Zahnprothese in Wasser eingelegt werden, woraufhin die Reinigungstablette dann unter mehr oder weniger starker Gasentwicklung zerfällt und Wirkstoffe freigibt, welche die auf der Zahnprothese anhaftenden Schmutzbeläge angreifen und entfernen sollen.

Derartige Reinigungstabletten, wie sie im übrigen auch Gegenstand der DE-PSn 23 12 847, 25 48 469, 26 58 450, und 27 41 072, in den EP-PSn 0 010 412, 0 028 005 und 0 102 418 sowie der EP-OS 0 133 354, 0 149 308, 0 157 464 und 0 225 658 sind, enthalten im wesentlichen sauerstoffabgebende Substanzen zum Oxidieren und Desinfizieren (wie Monopersulfate und/oder Perborate), anorganische Enthärter und Kalkbinder (wie Phosphate und/oder Polyphosphate, Chelatbildner, wie EDTA, gasbildende Träger, wie Carbonate und/oder Bicarbonate, Zitronensäure, Weinsäure, Amidosulfonsäure bzw. deren Salze, Desinfektionsmittel, wie Benzoate und/oder Hydroxybenzoesäurealkylester, sowie Gleit-, Binde- und/oder Sprengmittel, Tenside, Farbstoffzusätze und schließlich Aromamasse(n). Die pH-Werte bei Auflösung in Wasser schwanken, je nach Zusammensetzung, zwischen sauer, neutral und alkalisch. In den meisten Fällen handelt es sich dabei um Schnellreinigungstabletten, d. h. solche mit einer Reinigungszeit von 10 bis 15 Minuten; in einigen Fällen handelt es sich aber auch um langsam wirkende Tabletten mit relativ langen Anwendungszeiten, bei denen die volle Reinigungswirkung hinsichtlich der darin behandelten Zahnprothese erst in etwa 1 bis 2 Stunden erreicht wird.

Alle vorstehend beschriebenen Tabletten sind hinsichtlich der Reinigung von Zahnprothesen mit einem Nachteil behaftet, welcher für die Zusammensetzung und die Anwendungspraxis typisch ist: Die Bildung des Perborat-Sauerstoffes in der gewünschten aktiven Form über das Ion HOO- erfolgt zufriedenstellend nur bei Temperaturen oberhalb von 60 °C. Üblicherweise erfolgt die Reinigung von Zahnprothesen durch den Benutzer aber bei etwa 30 bis 40 °C in 10 bis 15 Minuten, so daß bei den bekannten Reinigungstabletten ein großer Teil der beabsichtigten oxidierenden sowie desinfizierenden Wirkung der sauerstoffabgebenden Substanzen nicht zur Geltung kommt.

Aus der EP-OS 0 253 772 ist eine Reinigungstablette der gattungsgemäßen Art bekannt, bei der als Phosphorverbindung eine Phosphatkombination, als solche den Reinigungsprozeß unterstützend bzw. hieran beteiligt, vorgesehen ist, während polymerer Fluorkohlenwasserstoff als Bindemittel verwendet wird. Bei der Verwendung eines derartigen hydrophoben Fluorkohlenwasserstoffs besteht ein Problem darin, daß er beim Auflösen der Reinigungstablette wegen seiner hydrophoben Natur nicht nur das Auflösen als solches erschwert, sondern darüber hinaus in der aufgelösten Reinigungsflotte in die Reaktionspartner trennender Weise und damit reinigungsverzögernd floatet. Die Verwendung eines derartigen polymeren, hydrophoben Bindemittels macht auch verständlich, warum bei der Reinigungstablette nach der EP-OS 0 253 772 ein hoher Aktivatorgehalt von 5 bis 15 Gew.-% verwendet wird, wobei dieser Anteil zu Lasten des Gehaltes an tatsächlich reinigungsspezifisch wirksamen Substanzen geht. Außerdem finden, wie bereits erwähnt, bei der bekannten Reinigungstablette anorganische Phosphate Verwendung, die ebenfalls wie das eingesetzte EDTA als Metall-Binder wirksam sind, jedoch wegen der möglichen Umwelteinflüsse als nicht unbedenklich gelten.

EP-A-0 323 049 offenbart eine Gebißreinigungstablette, die Natriumhydrogencarbonat, Zitronensäure, Perborat und Persulfat, Perborat/Peroxidaktivator in einer Menge von 0,5 bis 5 Gew.-% und fakultativ Tenside, Gleitmittel, Konservierungsstoffe, Aromastoffe sowie Komplexbildner enthält, wobei die Komplexbildner aus Phosphaten, Aminopolycarboxylaten oder 0,1-1 Gew.-% Phosphonsäuren oder deren Salzen ausgewählt sein können. In sämtlichen in den Beispielen der Druckschrift beschriebenen Gebißreinigungsmitteln sind Phosphate und/oder Aminocarboxylate enthalten.

Demgegenüber liegt der Erfindung die Aufgabe zugrunde, eine gattungsgemäße Reinigungstablette zur Verfügung zu stellen, welche, ohne die Stabilität und Wirksamkeit zu verringern, keine umweltbelastenden Phosphate und/oder Aminopolycarboxylate enthält.

Erfindungsgemäß wird diese Aufgabe durch die im Kennzeichen des Hauptanspruches aufgeführten Merkmale gelöst. Besonders bevorzugte Ausführungsformen der Erfindung sind Gegenstand der Unteransprüche.

Der Erfindung liegt die überraschende Erkenntnis zugrunde, daß es gelingt, die eingangs geschilderten Probleme der bekannten Reinigungstabletten dadurch zu überwinden, daß als Metall-Binder und zum Stabilisieren des Peroxids - und Verzicht auf ein hydrophobes polymeres Bindemittel - eine organische Phosphonsäure eingesetzt wird. Diese stellt gleichzeitig einen hydrolysestabilen Komplexbildner z. B. für

Alkalimetalle und Schwermetalle dar und ersetzt überdies die unter Umweltgesichtspunkten problematischen Phosphate/Polyphosphate sowie EDTA, die bei den bisher bekannten Reinigungsmitteln für Zahnprothesen die anorganischen Komplexbildner darstellen. Beim Auflösen der Reinigungstablette wird das Peroxid nicht durch ein inertes Polymeres behindert, sondern ist in der Reinigungstablette durch eine Substanz stabilisiert, welche beim Reinigungsprozeß nach dem Auflösen die Wirkung hat, durch Wegfangen der im Wasser vorhandenen Kontaktgifte etc. die Aktivatorwirkung systemimmanent von negativen Einflüssen freizuhalten. Es reicht daher erfindungsgemäß bereits ein Aktivatorgehalt von nur 0,5 bis 3,0 Gew.-% aus, wodurch der Anteil der Reinigungstablette an tatsächlich aktiven Substanzen weiter erhöht werden kann. Dies gelingt ferner auch dadurch, daß bei der Reinigungstablette nach der Erfindung wegen der hohen Wirksamkeit des Aktivators fünf bis zehn Teile Polyphosphate wie sie bei den Reinigungstabletten nach dem Stand der Technik gang und gäbe sind, durch nur ein Teil Phosphonate ersetzt werden können.

Das volle Wirksamwerden des Peroxids und Aktivators in der Reinigungsflotte, ohne darin floatendes "Fremdmaterial" in Form z. B. hydrophober Polymerer, gewährleistet, daß über die gesamte Reinigungsdauer von z. B. 15 Minuten trotz der dabei langsam absinkenden Temperatur der Reinigungsflotte in einem Wasserglas oder dgl. die volle Wirksamkeit der Peroxide erhalten wird, wie dies Gegenstand einer bevorzugten Ausführungsform der Erfindung ist, als (ein) Aktivator Tetraacetylethylendiamin (TAED) eingesetzt, so läßt sich die Abgabe von aktivem Sauerstoff in der für die reinigende und antimikrobielle Wirkung gewünschten Form über die Bildung der hochwirksamen Peressigsäure deutlich verstärken und stabilisieren.

Die erfindungsgemäß verwendeten hydrolysestabilen organischen Phosphonsäuren bzw. deren Salze weisen eine erhöhte kalk-, metall- und insbesondere eisenbindende Wirkung auf und wirken gleichzeitig dispergierend/entflockend. Die erfindungsgemäß bevorzugt vorgesehene Ausstattung der Reinigungstablette mit einem Farbstoffsystem der beanspruchten Art gibt die Möglichkeit, den Reinigungsablauf bis zum Ende der Hauptreinigung und auch der gesamten Reinigung optisch zu verfolgen. Im Laufe des Reinigungsprozesses folgt bei dieser Ausführungsform eine quantitative Abschwächung der Farbintensität, gefolgt von einem qualitativen Farbumschlag, bevor eine Entfärbung eintritt.

Charakteristisch ist es für die Reinigungstablette nach der Erfindung, daß sie keine umweltschädlichen anorganischen Phosphate, Polyphosphate sowie organisch Komplexbildner auf der Basis von Aminocarbonsäuren (z. B. EDTA) enthält. Die reinigende und antimikrobielle Wirkung ist im Bereich der praktischen Anwendung temperaturunempfindlich, d. h., die Tablette kann mit optimaler Reinigungswirkung im Bereich von ca. 25 °C bis ca. 45 °C eingesetzt werden.

Durch den bei der Reinigungstablette nach der Erfindung eingesetzten Perborataktivator wird in wässeriger Lösung ein Reaktionsmechanismus initiiert, der sich vereinfacht wie folgt darstellen läßt:

a) $$[B_2O_8H_4]^{2-} \cdot 2Na^+ \longrightarrow 2Na\,BO_2 + 2H_2O_2$$

Perboratmonohydrat — Wasserstoffperoxid

b) $$H_2O_2 + OH^- \rightleftharpoons H_2O + HOO^-$$

c) 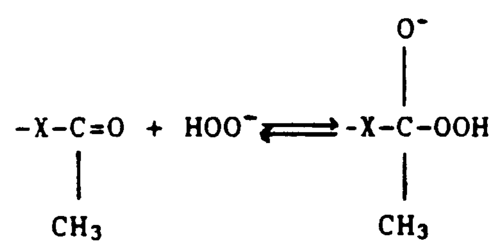

Acetylgruppe des Aktivators

d) 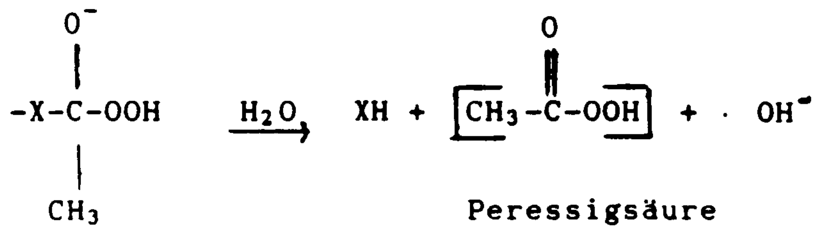

Peressigsäure

e) 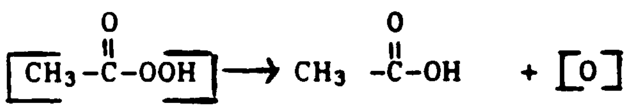

Essigsäure Aktiver Sauerstoff

Wichtig ist dabei, daß als Zwischenstufe Peressigsäure gebildet wird, die dann in Essigsäure und Sauerstoff zerfällt. Die so aktivierte Sauerstoffabgabe des Perborats führt zu einer deutlich verbesserten Reinigungswirkung.

Die über den Perborat/Peroxidaktivator, mit Peressigsäure als Zwischenstufe, erzielte antimikrobielle Wirkung der Reinigungstablette nach der Erfindung übertrifft ebenfalls die Erwartungen. Es zeigt sich, daß selbst bei hohen Verdünnungen und innerhalb kurzer Einwirkungszeiten Keimfreiheit erzielt wird, wobei die konkrete Zusammensetzung der Reinigungstablette in für den Fachmann geläufiger Weise entsprechend den jeweils erzielten Zwecken variierbar. Um nach 48-stündiger Bebrütung bakterienfreie Kulturen zu erhalten, genügt bei einer Peressigsäurekonzentration von 0,002 bis 0,005 Gew.-% eine Einwirkungszeit von 2 bis 10 Minuten an den üblichen Testbakterien (Escherichia coli, Staphylococcus aureus, Streptococcus mutans, Pseudomonas aeruginosa, Proteus vulgaris, Bacillus mesenterieus), um diese abzutöten. Dasselbe gilt auch für Faden- und Sproßpilze (Trichophyton rubrum, Candida albicans). Selbst bei den als resistent bekannten Enteroviren wird bei der erfindungsgemäßen Reinigungstablette eine mehrfach geringere Konzentration benötigt, infolge der vorhandenen Peressigsäure, als bei dem bisher eingesetzen $H_2O_2$.

Während die Rezepturen der meisten bekannten Reinigungsmittel für Zahnprothesen anorganische Phosphate und Polyphosphate zur Kalkbildung und Dispergierung sowie organische Komplexbildner auf der Basis von Aminocarbonsäure, wie EDTA und NTA, enthalten, benötigt die Reinigungstablette nach der Erfindung derartige Zusätze nicht. Dies ist von Vorteil, weil die Polyphosphate hydrolyseempfindlich sind, d. h., sie hydrolysieren in wässeriger Lösung unter Bildung von Orto-Phosphaten und verlieren dadurch ihre Dispergierwirkung, während EDTA vornherein keine Dispergierwikung hat. Des weiteren ist festzuhalten, daß anorganische Phosphate und z. B. EDTA unter Umweltgesichtspunkten nicht unbedenklich sind, da die einen durch die Anreicherung des Vorfluters mit sauerstoffverzehrenden Pflanzen, die anderen durch die schwere Abbaubarkeit Probleme mit sich bringen.

Bei der erfindungsgemäßen Reinigungstablette werden beide vorgenannten, unter Umweltgesichtspunkten nicht unproblematischen Produktgruppen aus der Gesamtzusammensetzung ferngehalten. Die bei Reinigungstabletten für Zahnprothesen erfindungsgemäß erstmals eingesetzten hochwirksamen Kalk- und Metallbinder in Form von organischen Komplexbildnern auf der Basis von Phosphonsäure bzw. der Salze sind hydrolysestabil, zeigen eine korrosionshindernde Wirkung und verbessern den Reinigungsvorgang zusätzlich dadurch, daß sie in synergistischem Zusammenwirken mit dem Perborataktivator den Sauerstoffabgeber bzw. die Sauerstoffabgabe stabilisieren und gleichzeitig gute dispergierende und entflockende Eigenschaften haben.

Praktisch alle bekannten bzw. marktüblichen Reinigungstabletten für Zahnprothesen schreiben für die Reinigungsflotte warmes Wasser, einige bis 50 °C, vor. Bei den meisten herkömmlichen Rezepturen ist dies auch unbedingt erforderlich, um den aktiven Sauerstoff des Peroxids rechtzeitig in Lösung zu bringen. Dabei müssen jedoch anderweitige Nachteile in Kauf genommen werden, z. B. schnellere Hydrolyse, schwächere Dispergierung/Entflokkung, begrenzte Korrosionsverhinderung. Als Folge der erfindungsgemäßen Wirkung des Peroxidaktivators/Stabilisators und der hydrolysenstabilen organischen Komplexbildner auf der Basis von Phosphonsäure sowie durch Eliminierung der hydrolyseempfindlichen anorganischen Polyphosphate weist die erfindungsgemäße Reinigungstablette die oben beschriebene Nachteile nicht auf. Außerdem ist sie auch ab Zimmertemperatur und nicht nur bei den bisher für die Praxis notwendigen

Warmwassertemperaturen voll wirksam. Für den Benutzer entfällt daher die bislang berechtigte Sorge um die Reinigungs- und Desinfektionswirkung bei mangelhafter Verfolgung der Temperatur der herstellerseitig empfohlenen warmen Reinigungsflotte.

Bei den bekannten Reinigungstabletten ist eine einwandfreie sensorische/visuelle Verfolgung des Ablaufs und des Endes des Reinigungsprozesses bislang nicht möglich. Selbst bei den bekannten Zweischichttabletten ist lediglich eine Vorreinigungs-Zwischenstufe erkennbar, während der Abschluß der Haupt- und Endreinigung dem Benutzer verborgen bleibt. Die bisher verwendeten Farben der Tabletten (z. B. blau, rosa) sind bei Verwendung von Glasgefäßen auch lange nach Beendigung des Waschvorganges noch erkennbar, selbst wenn ein anderer Effekt an sich angestrebt ist.

Wird hingegen das erfindungsgemäß vorgeschlagene Farbstoffsystem verwendet, so lassen sich die bislang bezüglich der optischen Verfolgung des Reinigungsprozesses bestehende Probleme beseitigen. Dabei handelt es sich um die Anwendung eines redoxempfindlichen Farbstoffes mit einem relativ schwachen Oxydationspotential in Kombination mit einem von Redoxpotential unabhängigen anders farbigen Farbstoff. Die durch die Oxydation der Schmutzstoffe freigewordenen Protonen sowie eine gewisse Protonabgabe aus den anderen Tablettensubstanzen bewirken eine quantitative Verringerung der Farbstoffintensität, gefolgt von einem qualitativen Farbumschlag, bevor eine Entfärbung eintritt. Der zeitliche Verlauf ist vom Verschmutzungsgrad der Zahnprothese und teilweise auch von der Temperatur und dem pH-Wert des Wassers abhängig. Für praktisch alle Anwendungsfälle läßt sich jedoch das Ende der Hauptreinigungsphase durch den Farbumschlag und das Ende des für die vorgesehene Einwirkungszeit optimierten Reinigungsprozesses durch vollständige Entfärbung auch optisch verfolgbar machen. Dies kann erfindungsgemäß durch verschiedene einschlägige Farbsysteme bewerkstelligt werden, wobei bei Verwendung der hier vorgesehenen Kombination mit Indigotindisulfonsäure, Dinatriumsalz und Chinophthalondisulfonsäure, Dinatriumsalz, eine grün-türkis-Farbe ensteht. Die Farbintensität verringert sich im Laufe des Reinigungsprozesses und schlägt dann in grün-gelb um (Ende der Hauptreinigung), bevor die Entfärbung erfolgt (Ende der Reinigung).

Die Reinigungstablette nach der Erfindung kann in folgenden Zusammensetzungsbereichen aufgebaut werden:

| | |
|---|---|
| Natriumperboratmonohydrat | 15 - 30 Gew.-% |
| Kaliummonopersulfat | 20 - 40 Gew.-% |
| Natriumhydrogencarbonat | 10 - 30 Gew.-% |
| Natriumcarbonat | 0 - 10 Gew.-% |
| Natriumsulfat | 0 - 10 Gew.-% |
| Zitronensäure bzw. Citratsalze sowie andere ähnlich wirkende organische Säuren bzw. deren Salze | 5 - 20 Gew.-% |
| Tetraacetylethylendiamin sowie andere ähnlich wirkende Peroxidaktivatoren | 0,3 - 3 Gew.-% |
| organische Phosphonsäure bzw. deren Salze | 0,5 - 3 Gew.-% |
| Polyethylenglykol 20.000 | 1 - 4,5 Gew.-% |
| Polyvinylpyrrolidon | 0 - 3 Gew.-% |
| Siliciumdioxid (Aerosil 200/300) | 0,5 - 1,5 Gew.-% |

Natriumdodecylbenzolsulfonat
Ricinylmonoethanolamid-sulfosuccinat, Dinatrium Salz

| | |
|---|---|
| oder ähnlich wirkende Tenside | 0,2 - 1,5 Gew.-% |
| Gehärtete Triglyceride | 0 - 2 Gew.-% |
| Fettalkoholpolyglykolaether | 0,5 - 2 Gew.-% |
| Konservierungsmittel, gleichzeitig Stabilisator | 0 - 2 Gew.-% |
| Pfefferminzpulver bzw. andere Aromastoffe (z. B. Eukalyptus) | 1 - 2 Gew.-% |
| Farbstoffsystem (z. B. Indigotin L-Blue 2 und Chinolingelb L-gelb 3) | 0,05 - 0,35 Gew.-% |

Nachstehend ist eine Reinigungstablette nach der Erfindung anhand eines konkreten Ausführungsbeispieles beschrieben:
30 Gew.-% Natriumperboratmonohydrat
20 Gew.-% Kaliummonopersulfat
20 Gew.-% Natriumhydrogencarbonat
5 Gew.-% Natriumcarbonat
4 Gew.-% Natriumsulfat
7 Gew.-% Zitronensäure, Natriumsalz
1,5 Gew.-% Tetraacethylendiamin
1,5 Gew.-% organische Phosphonsäuren bzw. deren Salze
4 Gew.-% Polyethylenglykol 20.000
1,5 Gew.-% Polyvinylpyrrolidon
1,5 Gew.-% Aerosil 200/300
0,75 Gew.-% Natriumdodecylbenzolsulfonat
0,5 Gew.-% Gehärtete Triglyceride
1 Gew.-% Fettalkoholpolyglykolaether
1 Gew.-% Konservierungsmittel
0,5 Gew.-% Pfefferminzpulver, und
0,25 Gew.-% Indigotin L-Blue 2 und Chinolingelb L-gelb 3,
wurden als Pulver bzw. in granulierter Form in gleichen Anteilen zu einer Reinigungstablette mit bekannten Techniken verpreßt. Die so entstandene Reinigungstablette zeigte nach dem Auflösen einen pH-Wert in der Reinigungsflotte von 9,0, bei einer Temperatur der Reinigungsflotte von 30 °C, wobei der Reinigungsprozeß, in seinem Ablauf voll optisch verfolgbar durch entsprechenden Farbumschlag, nach etwa 10 Minuten vollständig abgeschlossen war.

## Patentansprüche

1. Reinigungstablette zum selbsttätigen Reinigen von Zahnprothesen in wässeriger Lösung, mit einem Gehalt an Natriumhydrogencarbonat und/oder Natriumcarbonat(en), Zitronensäure bzw. Zitrat(en), Natriumperborat, Kaliummonopersulfat, Tensiden und Schaumbildnern, Binde-, Gleit- und Sprengmittel, antimikrobiellen Mitteln, Träger- und Aromastoffen, mindestens einem Perborat/Peroxid-Aktivator und mindestens einer Phosphorverbindung, dadurch gekennzeichnet, daß der Aktivatorgehalt zwischen 0,5 und 3,0 Gew.-% der Gesamtzusammensetzung der Reinigungstablette beträgt; daß als Phosphorverbindung ausschließlich mindestens eine als Stabilisierungsmittel wirkende hydrolysestabile organische

Phosphonsäure und/oder ein Salz derselben mit einem Gehalt zwischen 0,5 und 3,0 Gew.-% der Gesamtzusammensetzung der Reinigungstablette eingesetzt ist; und daß die Gesamtzusammensetzung der Reiningstablette von anorganischen Phosphaten/Polyphosphaten sowie organischen Komplexbildnern auf der Basis von Aminocarbonsäuren frei ist.

**2.** Reinigungstablette nach Anspruch 1, dadurch gekennzeichnet, daß als (ein) Aktivator Tetraacetylethylendiamin (TAED) eingesetzt ist.

**3.** Reinigungstablette zum selbsttätigen Reinigen von Zahnprothesen in wässeriger Lösung, mit einem Gehalt an Natriumhydrogencarbonat und/oder Natriumcarbonat(en), Zitronensäure bzw. Zitrat(en), Natriumperborat, Kaliummonopersulfat, Tensiden und Schaumbildner, Binde-, Gleit- und Sprengmitteln, antimikrobiellen Mitteln, Träger- und Aromastoffen, nach einem der vorangehenden Ansprüche, gekennzeichnet durch einen Gehalt an einem Farbstoffsystem mit mindestens zwei Farbstoffen, von denen mindestens einer in der mit chromophoren Gruppen gefärbten Keto-Form vorliegt und durch die während der Reinigung freiwerdenden Wasserstoffionen in die farblose desmotropische Leuco-enol-Form übergeht und mindestens einer redoxneutral ist.

**4.** Reinigungstablette nach Anspruch 3, dadurch gekennzeichnet, daß das Farbstoffsystem eine Kombination von Natrium-Indigotindisulfonat und Natrium- Chinophthalondisulfonat aufweist.

**5.** Reinigungstablette nach Anspruch 3 oder 4, dadurch gekennzeichnet, daß der Farbstoffsystemgehalt der Gesamtzusammensetzung der Reinigungstablette zwischen 0,05 und 0,35 Gew.-% beträgt.

**6.** Reinigungstablette nach einem der vorangehenden Ansprüche, dadurch gekennzeichnet, daß der pH-Wert einer damit durch Auflösen in Wasser hergestellten Reinigungsflotte zwischen 7,6 und 9,6 beträgt.

**7.** Reinigungstablette nach einem der vorangehenden Ansprüche, gekennzeichnet durch einen mehrschichtigen Aufbau.

**8.** Reinigungstablette nach einem der vorangehenden Ansprüche, dadurch gekennzeichnet, daß der Gehalt der Gesamtzusammensetzung der Reinigungstablette an der Phosphorverbindung zwischen 0,5 und 2,5 Gew.-% beträgt.

**Claims**

**1.** Cleaning tablet for the automatic cleaning of dentures in an aqueous solution, with a content of sodium hydrogen carbonate and/or sodium carbonate or carbonates, citric acid or citrate or citrates, sodium perborate, potassium monopersulphate, surfactants and foam forming agents, binders, lubricants and disintegrants, antimicrobial agents, carriers, flavouring/aromatising substances, at least one perborate/peroxide activator and at least one phosphorus compound, characterised in that the activator content is between 0.5 and 3.0% by weight of the total composition of the cleaning tablet, that the phosphorus compound used is exclusively at least one hydrolysis-stable organic phosphonic acid and/or a salt thereof acting as a stabilising agent and with a content between 0.5 and 3.0% by weight of the total composition of the cleaning tablet and that the total composition of the cleaning tablet is free from inorganic phosphates/polyphosphates and organic sequestrants based on aminocarboxylic acids.

**2.** Cleaning tablet according to claim 1, characterised in that tetraacetyl ethylene diamine (TAED) is used as the or an activator.

**3.** Cleaning tablet for the automatic cleaning of dentures in aqueous solution, with a content of sodium hydrogen carbonate and/or sodium carbonate or carbonates, citric acid or citrate or citrates, sodium perborate, potassium monopersulphate, surfactants and foam forming agents, binders, lubricants and disintegrants, antimicrobial agents, carriers, aromatising/flavoring substances, according to one of the preceding claims, characterised by a content of a dye system with at least two dyes whereof at least one is present in the keto-form coloured with chromophoric groups and as a result of the hydrogen ions released during cleaning passes into the colourless, desmotropic leuco-enol form and at least one is redox-neutral.

4. Cleaning tablet according to claim 3, characterised in that the dye system has a combination of sodium-indigotin disulphonate and sodium quinophthalone disulphonate.

5. Cleaning tablet according to claim 3 or 4, characterised in that the dye system content in the total composition of the cleaning tablet is between 0.05 and 0.35% by weight.

6. Cleaning tablet according to one of the preceding claims, characterised in that the pH value of a cleaning liquid produced therewith by dissolving in water is between 7.6 and 9.6.

7. Cleaning tablet according to one of of the preceding claims, characterised by a multilayer structure.

8. Cleaning tablet according to one of the preceding claims, characterised in that the phosphorus compound content of the total cleaning tablet composition is between 0.5 and 2.5% by weight.

**Revendications**

1. Comprimé de nettoyage pour le nettoyage spontané de prothèses dentaires en solution aqueuse, contenant du bicarbonate de sodium et/ou du carbonate de sodium, de l'acide citrique ou des citrates, du perborate de sodium, du monopersulfate de potassium, des agents tensio-actifs et agents moussants, des liants, lubrifiants et agents désagrégeants, des agents antimicrobiens, des véhicules et des substances aromatiques, au moins un activateur du perborate et des peroxydes et au moins un dérivé du phosphore, caractérisé en ce que la teneur en activateur représente de 0,5 à 3,0 % en poids de la composition globale du comprimé de nettoyage ; en ce que l'on utilise exclusivement, en tant que dérivé du phosphore, au moins un acide organique phosphonique et/ou l'un de ses sels, stable à l'hydrolyse et agissant comme stabilisant, à une teneur de 0,5 à 3,0 % du poids de la composition globale du comprimé de nettoyage ; et en ce que la composition globale du comprimé de nettoyage est exempte de phosphate/polyphosphates minéraux et d'agents complexants organiques à base d'acides aminocarboxyliques.

2. Comprimé de nettoyage selon la revendication 1, caractérisé en ce que l'on utilise au moins un activateur consistant en la tétracétyléthylènediamine (TAED).

3. Comprimé de nettoyage pour le nettoyage spontané de prothèses dentaires en solution aqueuse, contenant du bicarbonate de sodium et/ou du carbonate de sodium, de l'acide citrique ou des citrates, du perborate de sodium, du monopersulfate de potassium, des agents tensio-actifs et des agents moussants, des liants, des lubrifiants et des agents désagrégeants, des agents antimiicrobiens, des véhicules et des substances aromtiques selon une des revendications qui précèdent, caractérisé en ce qu'il contient un système colorant à au moins deux colorants dont l'un au moins est sous la forme céto colorée par des groupes chromophores et passe sous la forme desmotropique incolore leuco-énol sous l'action des ions hydrogènes libérés au cours du nettoyage, et l'un au moins est neutre à l'oxydoréduction.

4. Comprimé de nettoyage selon la revendication 3, caractérisé en ce que le système colorant contient une combinaison d'indigotine-disulfonate de sodium et de quinophtalone-disulfonate de sodium.

5. Comprimé de nettoyage selon la revendication 3 ou 4, caractérisé en ce que le système colorant est présent à une teneur de 0,05 à 0,35 % du poids de la composition globale du comprimé de nettoyage.

6. Comprimé de nettoyage selon l'une des revendications qui précèdent, caractérisé en ce que le pH d'un bain de nettoyage préparé par dissolution du comprimé dans l'eau est de 7,6 à 9,6.

7. Comprimé de nettoyage selon l'une des revendications qui précèdent, caractérisé en ce qu'il a une structure à plusieurs couches.

8. Comprimé de nettoyage selon l'une des revendications qui précèdent, caractérisé en ce que la teneur du comprimé de nettoyage en le dérivé du phosphore est de 0,5 à 2,5 % du poids de la composition globale.